# EUROPEAN PATENT APPLICATION

(11) **EP 4 227 961 A1**
(43) Date of publication of application: **16.08.2023**
(21) Application number: 23156382.6
(22) Date of filing: 13.02.2023
(51) Int. Cl.: G16H 50/20, G16B 40/00, G16H 30/20, G16H 30/40

(54) **MEDICAL INFORMATION PROCESSING APPARATUS AND COMPUTER PROGRAM**

(30) Priority: 15.02.2022 JP 2022021542
(71) Applicant: Canon Medical Systems Corporation, Tochigi 324-0036 (JP)
(72) Inventor: WATANABE, Kohei, Otawara-shi, 324-0036 (JP); MORI, Kei, Otawara-shi, 324-0036 (JP); FUJITO, Tomoki, Otawara-shi, 324-0036 (JP)
(74) Representative: Marks & Clerk LLP

(57) **Abstract**

A medical information processing apparatus according to an embodiment includes an acquisition unit, a determination unit, and a reception unit. The acquisition unit acquires an examination value of a biomarker collected from an examination target subject. The determination unit determines a first disease type of a disease based on an inference result of a first trained model, the inference result being obtained by inputting the acquired examination value the first trained model, the first trained model being configured to infer the first disease type of an affected disease upon inputting of the examination value. The reception unit receives input of an examination result. The determination unit determines, when the examination result of the subject defies the first disease type, a second disease type of the disease with which the subject is potentially affected by using a second trained model in accordance with the defied first disease type.

## Description

### FIELD

Embodiments described herein relate generally to a medical information processing apparatus and a computer program.

### BACKGROUND

Recently, liquid biopsy, which is an examination that performs a disease diagnosis and the like by using body fluid such as blood, has been attracting attention as an examination with a small load on a subject such as a patient. A technology known as liquid biopsy determines the existence and type of a disease based on a detection result of a biomarker such as protein or gene.

### SUMMARY OF THE INVENTION

A medical information processing apparatus includes: an acquisition unit configured to acquire an examination value of a biomarker collected from an examination target subject; a determination unit configured to determine a first disease type of a disease with which the subject is potentially affected based on an inference result of a first trained model, the inference result being obtained by inputting the examination value acquired by the acquisition unit to the first trained model, the first trained model being configured to infer the first disease type of an affected disease upon inputting of the examination value; and a reception unit configured to receive input of an examination result related to the first disease type of the subject and obtained through examination by a method different from the biomarker. The determination unit determines, when the examination result of the subject defies the first disease type, a second disease type of the disease with which the subject is potentially affected by using a second trained model in accordance with the defied first disease type.

The determination unit may determine the second disease type of the disease with which the subject is potentially affected by using the second trained model obtained by excluding an element related to inference of the first disease type from the first trained model.

The determination unit may determine the second disease type of the disease with which the subject is potentially affected by using the second trained model subjected to learning with an examination value except for an examination value of a subject affected with a disease of the first disease type among examination values of biomarkers collected from a plurality of subjects.

The second trained model may be a trained model subjected to learning with an examination value except for an examination value of a subject affected with a disease of the first disease type among examination values of biomarkers collected from a plurality of subjects.

The second trained model may be a trained model subjected to learning with an examination value of a kind of biomarker except for an examination value of a kind of biomarker corresponding to a disease of the first disease type among examination values of a plurality of kinds of biomarkers collected from a plurality of subjects.

The determination unit may determine whether the subject is affected with the disease based on an inference result of a third trained model, the inference result being obtained by inputting the examination value acquired by the acquisition unit to the third trained model, the third trained model being configured to output existence of the disease as an inference result upon inputting of the examination value, and when having determined that the subject is affected with the disease, the determination unit may input the examination value acquired by the acquisition unit to the first trained model.

A medical information processing apparatus includes: an acquisition unit configured to acquire an examination value of a biomarker collected from an examination target subject; a determination unit configured to determine a first disease type of a disease with which the subject is potentially affected based on an inference result of a trained model, the inference result being obtained by inputting the examination value acquired by the acquisition unit to the trained model, the trained model being configured to infer the first disease type of an affected disease upon inputting of the examination value; and a reception unit configured to receive input of an examination result related to the first disease type of the subject and obtained through examination by a method different from the biomarker. The determination unit determines, when the examination result of the subject defies the first disease type, a second disease type of the disease with which the subject is potentially affected based on an inference result obtained by providing change in accordance with the defied first disease type to examination values of a plurality of kinds of biomarkers collected from the subject and inputting the examination values provided with the change to the trained model.

The determination unit may apply the change by invalidating an examination value of a kind of biomarker related to a disease of the defied first disease type among examination values of a plurality of kinds of biomarkers collected from the subject.

When the examination result of the subject defies the second disease type, the determination unit may newly determine the second disease type based on an inference result obtained by inputting, to the trained model, an examination value obtained by invalidating an examination value of a kind of biomarker related to diseases of the first disease type and the second disease type among examination values of a plurality of kinds of biomarkers collected from the subject.

The disease may be cancer.

A non-transitory computer readable medium includes instructions that cause a computer to execute: acquiring an examination value of a biomarker collected from an examination target subject; determining a first disease type of a disease with which the subject is potentially affected based on an inference result of a first trained model, the inference result being obtained by inputting the acquired examination value to the first trained model, the first trained model being configured to infer the first disease type of an affected disease upon inputting of the examination value; receiving input of an examination result related to the first disease type of the subject and obtained through examination by a method different from the biomarker; and determining, when the examination result of the subject defies the first disease type, a second disease type of the disease with which the subject is potentially affected by using a second trained model in accordance with the defied first disease type.

A non-transitory computer readable medium includes instructions that cause a computer to execute: acquiring an examination value of a biomarker collected from an examination target subject; determining a first disease type of a disease with which the subject is potentially affected based on an inference result of a first trained model, the inference result being obtained by inputting the acquired examination value at the acquiring to the first trained model, the first trained model being configured to infer the first disease type of an affected disease upon inputting of the examination value; receiving input of an examination result related to the first disease type of the subject and obtained through examination by a method different from the biomarker; and determining, when the examination result of the subject defies the first disease type, a second disease type of the disease with which the subject is potentially affected based on an inference result obtained by providing change in accordance with the defied first disease type to examination values of a plurality of kinds of biomarkers collected from the subject and inputting the examination values provided with the change to the first trained model.

### BRIEF DESCRIPTION OF THE DRAWINGS

FIG. 1 is a diagram illustrating an exemplary configuration of a medical information processing apparatus according to a first embodiment;
FIG. 2 is a diagram illustrating an example of a first determination model according to the first embodiment;
FIG. 3 is an explanatory diagram illustrating an example of a method of generating the first determination model according to the first embodiment;
FIG. 4 is a diagram illustrating an example of the result of tumor incidence site prediction by a determination function according to the first embodiment;
FIG. 5 is a diagram illustrating an example of the result of tumor incidence site prediction for a breast cancer patient by the determination function according to the first embodiment;
FIG. 6 is a flowchart illustrating an example of processing executed by the medical information processing apparatus according to the first embodiment;
FIG. 7 is a diagram illustrating an exemplary configuration of a medical information processing apparatus according to a second embodiment; and
FIG. 8 is a flowchart illustrating an example of processing executed by the medical information processing apparatus according to the second embodiment.

### DETAILED DESCRIPTION

A medical information processing apparatus according to an embodiment includes an acquisition unit, a determination unit, and a reception unit. The acquisition unit acquires an examination value of a biomarker collected from an examination target subject. The determination unit determines a first disease type of a disease with which the subject is potentially affected based on an inference result of a first trained model, the inference result being obtained by inputting the examination value acquired by the acquisition unit to the first trained model, the first trained model being configured to infer the first disease type of an affecting disease upon inputting of the examination value. The reception unit receives input of an examination result related to the first disease type of the subject and obtained through examination by a method different from the biomarker. The determination unit determines, when the examination result of the subject defies the first disease type, a second disease type of the disease with which the subject is potentially affected by using a second trained model in accordance with the defied first disease type.

### First embodiment

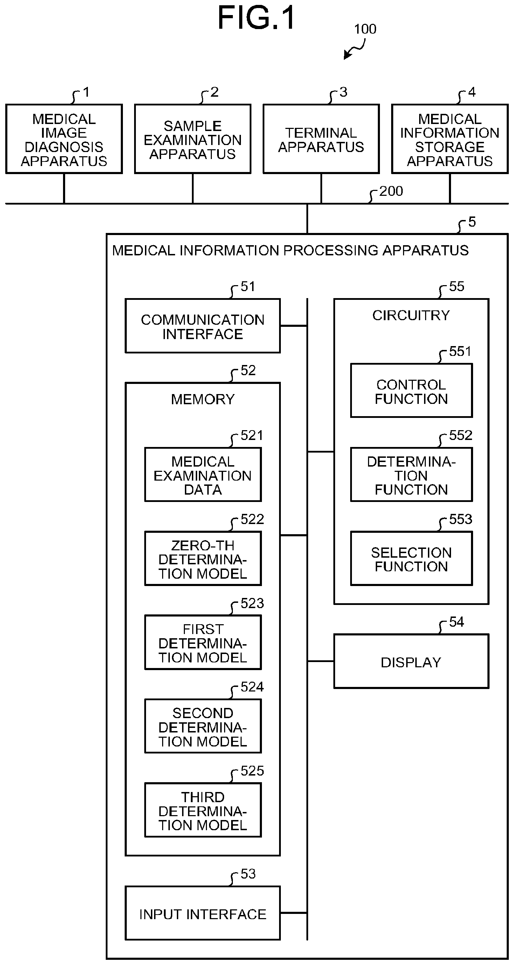

FIG. 1 is a block diagram illustrating an example of the configuration of a medical information processing apparatus 5 according to a first embodiment. For example, as illustrated in FIG. 1, the medical information processing apparatus 5 according to the first embodiment is included in a medical information processing system 100 in which the medical information processing apparatus 5 is connected to a medical image diagnosis apparatus 1, a sample examination apparatus 2, a terminal apparatus 3, and a medical information storage apparatus 4 to perform communication therebetween through a network 200.

The apparatuses included in the medical information processing system 100 can directly or indirectly communicate with one another through, for example, an in-hospital local area network (LAN) installed in a hospital. The medical information processing system 100 illustrated in FIG. 1 may be connected to a non-illustrated apparatus to perform communication therebetween.

For example, the medical information processing system 100 may include various kinds of systems such as a hospital information system (HIS), a radiology information system (RIS), a diagnosis report system, a picture archiving and communication system (PACS), and a laboratory information system (LIS).

The medical image diagnosis apparatus 1 collects a medical image by capturing an image of a subject. Then, the medical image diagnosis apparatus 1 transmits the collected medical image to the terminal apparatus 3, the medical information storage apparatus 4, and the medical information processing apparatus 5. The medical image diagnosis apparatus 1 is, for example, an X-ray diagnosis apparatus, an X-ray computed tomography (CT) apparatus, a magnetic resonance imaging (MRI) apparatus, an ultrasonic diagnosis apparatus, a single photon emission computed tomography (SPECT) apparatus, or a positron emission computed tomography (PET) apparatus.

The sample examination apparatus 2 is operated by a clinical laboratory technician or the like and executes sample examination that analyzes a sample such as blood or urine acquired from a patient. The sample examination is, for example, pathology examination, blood and biochemical examination, liquid biopsy examination, general examination of urine, feces, and the like, immunoserological examination, gene examination, microorganism examination, or examination related to blood transfusion and organ transplant. Then, the sample examination apparatus 2 transmits an examination result (such as measurement data) of the sample examination to the terminal apparatus 3, the medical information storage apparatus 4, and the medical information processing apparatus 5.

The sample examination apparatus 2 is, for example, a biochemical automatic analysis apparatus, an immunity automatic analysis apparatus, a flow cytometer, a gene analysis apparatus, a protein analysis apparatus, an extracellular vesicle analysis apparatus, or a circulating tumor cell detection apparatus. The flow cytometer is an instrument configured to perform flow cytometry. The flow cytometry is an analysis method of measuring, for example, the size and amount of a measurement target object by measuring scattering light and fluorescence generated by irradiating high-speed fluid as a suspension of the measurement target object with a laser beam, mercury light, or the like.

The gene analysis apparatus is an apparatus configured to analyze gene sequences. The gene analysis apparatus is, for example, an apparatus configured to amplify nucleic acid molecules extracted from a living body specimen by a polymerase chain reaction (PCR) method or the like and determine the existence and quantity of a particular gene sequence, or an apparatus configured to analyze sequence information of nucleic acid molecules. The protein analysis apparatus is, for example, an automatic immunoassay analysis apparatus or a highly-sensitive protein detection apparatus (for example, Single Molecule Assay SIMOA (registered trademark) manufactured by Quanterix).

The extracellular vesicle analysis apparatus is, for example, a single extracellular vesicle analysis apparatus (for example, EXOVIEW (registered trademark) manufactured by NanoView Biosciences). The circulating tumor cell detection apparatus is, for example, a circulating tumor cell detection apparatus by a nano magnetic bead method (for example, a CELLSEARCH (registered trademark) system manufactured by Veridex).

The terminal apparatus 3 is an apparatus operated by a doctor or a laboratory technician working in the hospital. The terminal apparatus 3 is achieved by, for example, a personal computer (PC), a tablet PC, a personal digital assistant (PDA), or a cellular phone.

The terminal apparatus 3 displays a medical image received from the medical image diagnosis apparatus 1, the sample examination apparatus 2, or the medical information storage apparatus 4 on a display of the own apparatus and receives various operations through an input interface of the own apparatus. For example, the terminal apparatus 3 receives operations to input various kinds of examination results, diagnosis information, and the like and transmits the input to the medical information storage apparatus 4 and the medical information processing apparatus 5.

The medical information storage apparatus 4 stores therein various kinds of medical information in the medical information processing system 100. Specifically, the medical information storage apparatus 4 stores therein medical information including medical images received from the medical image diagnosis apparatus 1, measurement data received from the sample examination apparatus 2, various kinds of examination results and diagnosis information received through the terminal apparatus 3, and various kinds of information received from any other apparatus connected to the medical information processing system 100.

The medical information storage apparatus 4 stores therein each piece of information included in the medical information in association with a patient ID and the like. For example, the medical information storage apparatus 4 is achieved by a computer device such as a database (DB) server and stores the medical information in a semiconductor memory device such as a random-access memory (RAM) or a flash memory and in a memory such as a hard disk or an optical disc.

Although FIG. 1 illustrates one medical information storage apparatus 4, the embodiment is not limited thereto and a plurality of DB servers may cooperatively function as the medical information storage apparatus 4.

The medical information processing apparatus 5 acquires various kinds of information from the medical image diagnosis apparatus 1, the sample examination apparatus 2, the terminal apparatus 3, and the medical information storage apparatus 4 and performs various kinds of information processing by using the acquired information. The medical information processing apparatus 5 is achieved by, for example, a computer device such as a server, a work station, a personal computer, or a tablet terminal.

As illustrated in FIG. 1, the medical information processing apparatus 5 includes a communication interface 51, a memory 52, an input interface 53, a display 54, and a circuitry 55.

The communication interface 51 is connected to the processing circuitry 55 and controls communication performed with each apparatus in the medical information processing system 100. Specifically, the communication interface 51 receives various kinds of information from each apparatus and outputs the received information to the processing circuitry 55. The communication interface 51 is achieved by, for example, a network card, a network adapter, a network interface controller (NIC).

The memory 52 is connected to the processing circuitry 55 and stores therein various kinds of data. Specifically, the memory 52 stores therein, for example, various kinds of information received from the medical image diagnosis apparatus 1, the terminal apparatus 3, and the medical information storage apparatus 4, information input through the input interface 53, and results of processing at the medical information processing apparatus 5. As illustrated in FIG. 1, the memory 52 stores therein, for example, medical examination data 521, a zero-th determination model 522, a first determination model 523, a second determination model 524, and a third determination model 525.

The medical examination data 521 includes medical images, analysis information of medical images, measurement data of the sample examination, and medical examination information that are received from the medical image diagnosis apparatus 1, the sample examination apparatus 2, the terminal apparatus 3, and the medical information storage apparatus 4. The medical examination data 521 is, for example, biomarker data. The biomarker data is the measurement value of a biomarker.

The biomarker is a material such as protein the concentration of which reflects the existence and stage of progression of a disease and that is measured in body fluid such as blood. Typically, the biomarker is an indicator of a particular state of disease or the state of a living organism. The biomarker is, for example, protein or gene, the blood concentration of which changes by reflecting the existence and stages of progression of various cancers such as breast cancer and colorectal cancer. The biomarker is detected by, for example, a flow cytometer or a gene analysis apparatus.

For example, FGFR1 gene and ESR1 gene detected as circulating tumor DNA (ctDNA), and CA15-3 as a tumor marker (protein) are known as biomarkers of breast cancer, NRAS gene and FBXW7 gene detected as ctDNA, and CA125 as a tumor marker are known as biomarkers of colorectal cancer, and MET gene and ALK gene detected as ctDNA, and CYFRA21-1 as a tumor marker are known as biomarkers of lung cancer.

The medical information processing apparatus 5 according to the present embodiment determines the type (tumor incidence site) of a tumor (cancer). The tumor (cancer) is an exemplary disease. The tumor incidence site is an exemplary disease type.

The medical examination data 521 is acquired from the medical image diagnosis apparatus 1, the sample examination apparatus 2, the terminal apparatus 3, and the medical information storage apparatus 4 through the communication interface 51 and stored in the memory 52. The medical examination data 521 is also acquired through processing at the processing circuitry 55 and stored in the memory 52.

The zero-th determination model 522, the first determination model 523, the second determination model 524, and the third determination model 525 are trained models generated by machine learning with the medical examination data 521 used as learning data.

Specifically, the zero-th determination model 522, the first determination model 523, the second determination model 524, and the third determination model 525 are generated by machine learning with medical examination data of each of a plurality of subjects and the disease type of the subject used as learning data and are stored in the memory 52. For example, a condition on the medical examination data 521 related to the generation is different among the zero-th determination model 522, the first determination model 523, the second determination model 524, and the third determination model 525.

The zero-th determination model 522 is used to determine the existence of a disease with which an examination target subject is affected based on the examination value of the biomarker of the subject. In the following description, the disease existence determination using the zero-th determination model 522 is also referred to as "zero-th determination".

The first determination model 523, the second determination model 524, and the third determination model 525 are used to determine a disease type based on the examination value of the biomarker of the examination target subject when it is determined that the subject is affected with a disease in the zero-th determination. In the following description, the disease type determination using the first determination model 523 is also referred to as "first determination", the disease type determination using the second determination model 524 is also referred to as "second determination", and the disease type determination using the third determination model 525 is also referred to as "third determination". The first determination model 523, the second determination model 524, and the third determination model 525 will be described later.

In the first embodiment, the zero-th determination model 522, the first determination model 523, the second determination model 524, and the third determination model 525 are generated by an external apparatus disposed outside the medical information processing system 100. However, the zero-th determination model 522, the first determination model 523, the second determination model 524, and the third determination model 525 may be generated by the processing circuitry 55.

The memory 52 also stores therein various kinds of computer programs configured to achieve various kinds of functions when the computer programs are read and executed by the processing circuitry 55. The memory 52 is achieved by, for example, a semiconductor memory device such as a random-access memory (RAM) or a flash memory, a hard disk, or an optical disc.

The input interface 53 is connected to the processing circuitry 55 and receives an operation to input various instructions and information from an operator. Specifically, the input interface 53 converts an input operation received from the operator into an electric signal and outputs the electric signal to the processing circuitry 55.

The input interface 53 is achieved by, for example, a track ball, a switch button, a mouse, a keyboard, a touch pad on which an input operation is performed through touch on an operation surface, a touch screen as an integration of a display screen and a touch pad, a non-contact input circuit using an optical sensor, or a voice input circuit.

In the present specification, the input interface 53 is not limited to that including a physical operation component such as a mouse or a keyboard. Examples of the input interface 53 include an electric signal circuitry that receives an electric signal corresponding to an input operation from an external input device provided separately from the apparatus and outputs the electric signal to a control circuit.

The display 54 is connected to the processing circuitry 55 and displays various kinds of information and images. Specifically, the display 54 converts information and image data transferred from the processing circuitry 55 into a display electric signal and outputs the display electric signal. The display 54 is achieved by, for example, a liquid crystal monitor, a cathode ray tube (CRT) monitor, or a touch panel.

The processing circuitry 55 controls operation of the medical information processing apparatus 5 in accordance with an input operation received from the operator through the input interface 53. The processing circuitry 55 is achieved by, for example, a processor. As illustrated in FIG. 1, the processing circuitry 55 executes a control function 551, a determination function 552, and a selection function 553. The control function 551 is an exemplary acquisition unit and an exemplary reception unit. The determination function 552 and the selection function 553 are an exemplary determination unit.

The control function 551 controls processing to be executed in accordance with various requests input through the input interface 53. The control function 551 controls, for example, transmission and reception of medical images, examination results, and the like through the communication interface 51, storage of various kinds of information in the memory 52, and display of information (such as medical images and results of processing by functions) on the display 54.

For example, the control function 551 acquires medical examination data from the medical image diagnosis apparatus 1, the sample examination apparatus 2, the terminal apparatus 3, and the medical information storage apparatus 4 and stores the medical examination data in the memory 52. Specifically, the control function 551 acquires, from the sample examination apparatus 2, the examination value of the biomarker collected from the examination target subject and stores the examination value as the medical examination data 521 in the memory 52.

For example, the control function 551 controls the display 54 to display a GUI for executing processing on the medical examination data, and a result of processing by each function.

The determination function 552 determines whether the target subject is affected with a disease based on an inference result of the zero-th determination model 522, the inference result being obtained by inputting the examination value acquired by the control function 551 to the zero-th determination model 522, the zero-th determination model 522 being configured to output the existence of a disease of the target subject upon inputting of the examination value.

Specifically, the determination function 552 inputs biomarker measurement data collected from the examination target subject to the zero-th determination model 522 subjected to learning with biomarker data of a plurality of subjects, and determines the existence of cancer (tumor) in the target subject based on the inference result of the zero-th determination model 522.

The following describes the zero-th determination model 522. The zero-th determination model 522 is a trained model configured to receive biomarker data and output the existence of a disease as an inference result. The zero-th determination model 522 uses, as learning data, biomarker data collected from a plurality of subjects and information indicating the existence of a disease of each subject.

Without using the zero-th determination model 522, the determination function 552 may determine the existence of a disease with which the subject is affected based on an inference result of the first determination model 523, the inference result being obtained by inputting the examination value acquired by the control function 551 to the first determination model 523.

When it is determined that a disease exists, the determination function 552 determines the disease type of a disease with which the subject is potentially affected based on an inference result of the first determination model 523, the inference result being obtained by inputting the examination value acquired by the control function 551 to the first determination model 523, the first determination model 523 being configured to infer the disease type of an affecting disease upon inputting of the examination value.

Specifically, when it is determined that a tumor exists in the zero-th determination, the determination function 552 inputs biomarker measurement data collected from the examination target subject to the first determination model 523 subjected to learning with biomarker data of a plurality of subjects and determines a cancer (tumor incidence site) with which the subject is potentially affected based on an inference result of the first determination model 523.

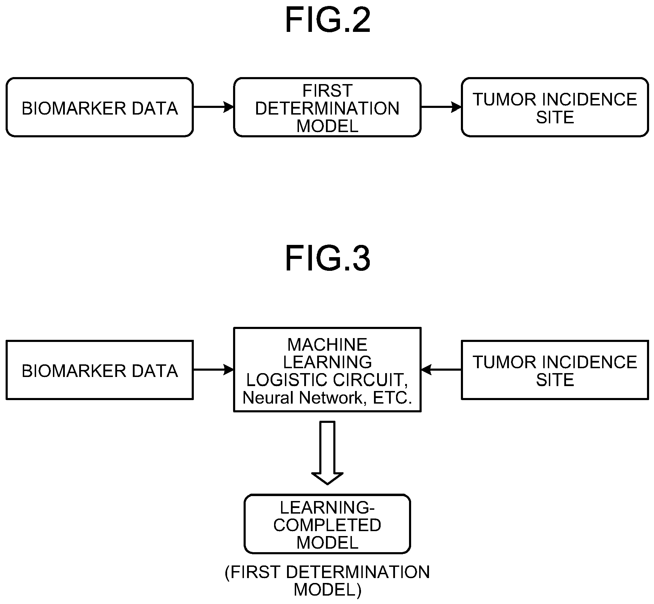

The following describes the first determination model 523 with reference to FIGS. 2 and 3. FIG. 2 is a diagram illustrating an example of the first determination model 523. As illustrated in FIG. 2, the first determination model 523 is a trained model configured to output, as an inference result, the disease type of a disease suspected to be affected upon inputting of biomarker data.

FIG. 3 is an explanatory diagram illustrating an example of the method of generating the first determination model 523. As illustrated in FIG. 3, the first determination model 523 uses, as learning data, biomarker data collected from a plurality of subjects and the disease type of each subject. The biomarker data as the learning data is preferably a plurality of pieces of biomarker data of different kinds such as "minor protein" and "gene".

In a case of a tumor, a disease type corresponds to the incidence site of the tumor and thus is referred to as "tumor incidence site" in FIG. 3. The "tumor incidence site" is information specified based on the result of a definitive diagnosis by biopsy or the like. Collected biomarker data of all subjects is used in learning of the first determination model 523 irrespective of the tumor incidence site.

The first determination model 523 having learned the relation between each of a plurality of pieces of biomarker data and a tumor incidence site can be generated by performing machine learning with the above-described learning data. Specifically, a trained model (first determination model) configured to output a tumor incidence site upon inputting of biomarker data is generated.

Specifically, the external apparatus (hereinafter also referred to as a learning apparatus) configured to generate a trained model inputs, as the learning data to a machine learning engine, biomarker data such as "minor protein" and "gene" of a plurality of subjects and their "tumor incidence sites" and causes the machine learning engine to perform machine learning. Then, the learning apparatus generates a trained model configured to output a tumor incidence site upon inputting of biomarker data.

The machine learning engine may be, for example, a neural network disclosed in a publicly known Non Patent Literature "Christopher M. Bishop: "Pattern recognition and machine learning", U.S.A., first edition, Springer, 2006, pp.225 to 290".

Alternatively, various algorithms such as deep learning, logistic regression analysis, non-linear discriminant analysis, support vector machine (SVM), random forest, and naive Bayes may be used in place of the above-described neural network as the machine learning engine.

As a result of such machine learning, the learning apparatus generates the first determination model 523 configured to output a numerical value indicating the probability of being affected as an inference result for each tumor incidence site upon inputting of "biomarker data (minor protein)", "biomarker data (gene)", ... to the trained algorithm.

In the present embodiment, the first determination model 523 outputs a numerical value indicating the probability of being affected as an inference result for each of breast cancer (mammary gland), colorectal cancer, liver cancer, lung cancer, ovary cancer, pancreas cancer, and upper gastrointestinal cancer (stomach and esophagus).

Then, the determination function 552 determines a cancer having a high affecting probability based on the inference result output from the first determination model 523. For example, when the inference result includes a cancer for which the numerical value indicating the probability of being affected exceeds a predetermined threshold, the determination function 552 determines the cancer as the cancer having a high affecting probability.

The determination function 552 may determine that no tumor exists when the threshold is not exceeded for any cancer. The determination function 552 may determine a cancer of the highest numerical value as the cancer having a high affecting probability. Alternatively, the determination function 552 may determine the cancer of the highest numerical value as the cancer having a high affecting probability only when the numerical value indicating the probability of being affected does not exceed the predetermined threshold for any cancer.

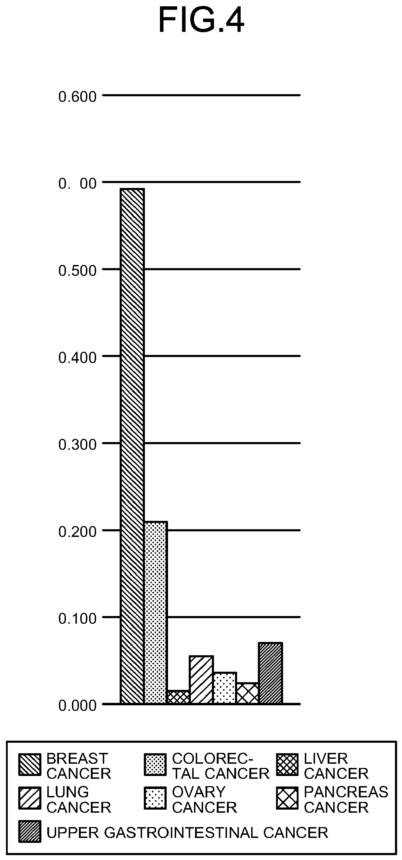

FIG. 4 is a diagram illustrating an example of the result of tumor incidence site inferred by the first determination model 523. As illustrated in FIG. 4, the first determination model 523 outputs the numerical value indicating the probability of being affected with a tumor as an inference result for each of breast cancer, colorectal cancer, liver cancer, lung cancer, ovary cancer, pancreas cancer, and upper gastrointestinal cancer such that the sum of the numerical values is equal to one. For example, when the cancer of the highest numerical value is determined as the cancer having a high affecting probability, the determination function 552 determines that a subject is highly likely to be affected with breast cancer in the example illustrated in FIG. 4.

In this case, detailed examination of breast cancer is performed for the subject. Detailed examination is examination by a method different from the biomarker. Detailed examination of breast cancer is, for example, mammography, ultrasound examination, MRI, or PET. Detailed examination of colorectal cancer is, for example, large intestine endoscopy or X-ray examination. Detailed examination of liver cancer is, for example, ultrasound examination, X-ray CT, or MRI.

Detailed examination of lung cancer is, for example, chest radiography, X-ray CT, or PET. Detailed examination of ovary cancer is, for example, ultrasound examination, X-ray CT, or MRI. Detailed examination of pancreas cancer is, for example, X-ray CT, MRI, ultrasound examination, or PET. Detailed examination of upper gastrointestinal cancer is, endoscopy, X-ray examination, X-ray CT, or PET.

The examination result of detailed examination is input through the terminal apparatus 3 by a medical professional such as a clinical laboratory technician. Then, the examination result is received by the control function 551.

For example, in the example illustrated in FIG. 4, the determination function 552 determines that the subject is highly likely to be affected with breast cancer, and thus mammography, ultrasound examination, MRI, PET, or the like is performed for the subject.

The medical professional inputs an examination result of detailed examination of breast cancer through the terminal apparatus 3. For example, when an examination result that breast cancer is detected is input by the medical professional, the control function 551 receives input of the examination result. Then, since the examination result of detailed examination affirms determination of breast cancer by the first determination, the determination function 552 confirms breast cancer as cancer with which the subject is highly likely to be affected.

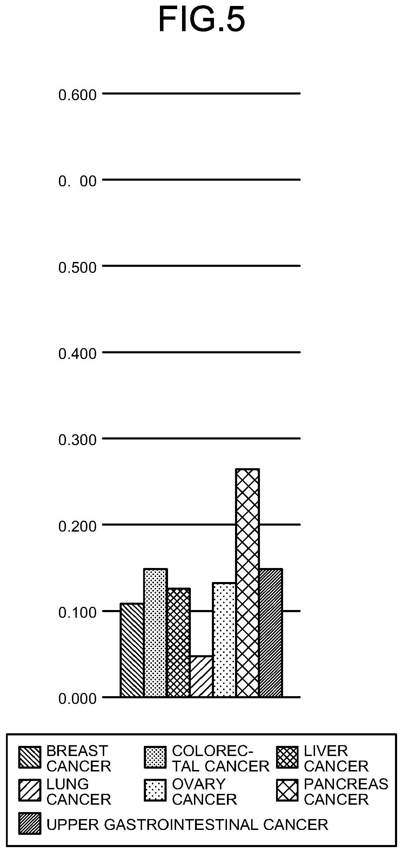

Depending on a tumor incidence site, the accuracy of determination by the first determination model 523 decreases and false determination is likely to occur. Thus, it is possible that no tumor is detected at a tumor incidence site of the highest probability through detailed examination. In such a case, detailed examination is performed for a tumor incidence site of the next highest probability, but the number of times of detailed examination potentially becomes extremely large depending on a result of tumor incidence site prediction. The following describes a case in which the number of times of detailed examination is large with reference to FIG. 5.

FIG. 5 is a diagram illustrating an example of the result of tumor incidence site determination by the determination function 552 for a breast cancer patient. For example, when the cancer of the highest numerical value is determined as the cancer having a high affecting probability, the determination function 552 determines that the subject is highly likely to be affected with pancreas cancer in the example illustrated in FIG. 5.

However, since the subject is a breast cancer patient, pancreas cancer is not detected by detailed examination. When detailed examination is to be performed in descending order of the probability of being affected, detailed examination of colorectal cancer, upper gastrointestinal cancer, ovary cancer, liver cancer, and breast cancer need to be further performed. When detailed examination is repeated in this manner, financial and physical loads on the subject increase. Thus, the number of times of detailed examination is preferably decreased as small as possible.

Thus, when the examination result of detailed examination of the subject defies the determination result of the first determination, the determination function 552 determines the disease type of a disease with which the subject is potentially affected by using the second determination model 524 in accordance with the defied result of the first determination.

For example, the determination function 552 determines the disease type of a disease with which the subject is potentially affected by using the second determination model 524 selected by the selection function 553 to be described later.

When an examination result that defies the determination result of the first determination is input by the control function 551, the selection function 553 selects a particular second determination model 524 to be used in the second determination from among a plurality of second determination models 524. The following describes the second determination models 524.

As an example, each second determination model 524 is a trained model obtained by excluding an element related to inference of a particular disease type from the first determination model 523. Specifically, the second determination model 524 is a trained model generated by using learning data except for an element related to inference of a particular disease type among learning data related to generation of the first determination model 523.

More specifically, the second determination model 524 is a trained model generated by inputting, to the machine learning engine, learning data of a subject except for learning data of a subject affected with a disease of a particular type among learning data of a plurality of subjects. The second determination model 524 is generated correspondingly for each disease type determined in the first determination.

The following describes processing of selecting a second determination model 524 by the selection function 553 in a case in which "tumor incidence site" is, for example, any of breast cancer, colorectal cancer, liver cancer, lung cancer, ovary cancer, pancreas cancer, and upper gastrointestinal cancer.

In this case, the second determination models 524 are seven trained models subjected to learning without biomarker data of a patient of one particular cancer, such as a trained model subjected to learning with biomarker data of a subject except for biomarker data of a patient of "breast cancer", and a trained model subjected to learning with biomarker data of a subject except for biomarker data of a patient of "colorectal cancer".

When the examination result of detailed examination of a subject, which is related to a disease type according to the first determination, defies the determination result of the first determination, the selection function 553 selects a second determination model 524 obtained by excluding an element related to inference of the disease type.

Description will be made with an exemplary case in which, for example, the first determination by the determination function 552 provides a result that the probability of "breast cancer" is high, and the control function 551 receives input of an examination result that no "breast cancer" is detected in detailed examination of "breast cancer".

In this case, the selection function 553 selects, as a second determination model 524 to be used in the second determination, the second determination model 524 subjected to learning with biomarker data of a subject except for biomarker data of a patient of "breast cancer" among the seven second determination models 524. Then, the determination function 552 performs the second determination by using the second determination model 524.

Since no data indicating "tumor incidence site" as "breast cancer" is included in learning data of the second determination model 524 subjected to learning with biomarker data of a subject except for biomarker data of a patient of "breast cancer", "breast cancer" is not output as "tumor incidence site" and it is not determined that the probability of "breast cancer" is high. Thus, there is no probability that "tumor incidence site" is falsely determined as "breast cancer" in the second determination by the determination function 552.

Moreover, since the determination function 552 uses, as a second determination model 524, a trained model subjected to learning with data except for data of a patient of "breast cancer", which is thought to be the cause of false determination in the first determination, the accuracy of determination in the second determination is expected to improve.

In the present embodiment, when the examination result of detailed examination of a subject, which is related to a disease type according to the first determination, defies the determination result of the first determination, the selection function 553 selects a second determination model 524 obtained by excluding an element related to inference of the disease type according to the first determination, but the selected second determination model 524 is not limited thereto.

For example, when the examination result of detailed examination of a subject, which is related to a disease type according to the first determination, defies the determination result of the first determination, the selection function 553 may select a second determination model 524 with a reduced weight for an element related to inference of the disease type according to the first determination.

The second determination model 524 in this case is, for example, a trained model subjected to learning with biomarker data made closer to a reference value by, for example, multiplying biomarker data of a patient of a particular cancer by a coefficient smaller than one (or a coefficient exceeding one).

When the examination results of detailed examination after the second determination and later determination defy a determined disease type, the determination function 552 newly determines a disease type by using a trained model obtained by excluding an element related to inference of the determined disease type from a trained model used in the determination of the disease type.

For example, when the control function 551 receives input of an examination result that no tumor is detected in detailed examination after the second determination, the determination function 552 performs the third determination by using the third determination model 525 selected by the selection function 553. The following describes the third determination model 525.

The third determination model 525 is a trained model obtained by excluding an element related to inference of a particular disease type from the second determination model 524. Specifically, the third determination model 525 is a trained model generated by using learning data obtained by excluding an element related to inference of a particular disease type from learning data related to generation of the second determination model 524.

More specifically, the third determination model 525 is a trained model generated by inputting, to the machine learning engine, learning data of a subject except for learning data of a subject affected with a particular disease type among the learning data of the second determination model 524. The third determination model 525 is generated correspondingly for each disease type determined in the second determination.

The following describes processing of selecting a third determination model 525 by the selection function 553 in a case in which "tumor incidence site" is, for example, any of breast cancer, colorectal cancer, liver cancer, lung cancer, ovary cancer, pancreas cancer, and upper gastrointestinal cancer.

In this case, the third determination models 525 are 21 trained models subjected to learning without biomarker data of patients of two particular cancers, such as a trained model subjected to learning with biomarker data of subjects except for biomarker data of patients of "breast cancer" and "colorectal cancer", and a trained model subjected to learning with biomarker data of subjects except for biomarker data of patients of "breast cancer" and "liver cancer".

When the examination result of detailed examination of a subject, which is related to a disease type according to the second determination, defies the determination result of the second determination, the selection function 553 selects a third determination model 525 obtained by excluding an element related to inference of the disease type.

Description will be made with an exemplary case in which, for example, the first determination by the determination function 552 provides a result that the probability of "breast cancer" is high, the control function 551 receives input of an examination result that no "colorectal cancer" is detected in detailed examination of "colorectal cancer", the second determination provides a result that the probability of "colorectal cancer" is high, and the control function 551 receives input of an examination result that no "colorectal cancer" is detected in detailed examination of "colorectal cancer".

In this case, the selection function 553 selects, as a second determination model 524 to be used in the third determination, a third determination model 525 subjected to learning with biomarker data of subjects except for biomarker data of patients of "breast cancer" and "colorectal cancer" among the 21 third determination models 525. Then, the determination function 552 performs the third determination by using the third determination model 525.

In the present embodiment, when no tumor is detected in detailed examination at the third determination, the determination function 552 confirms a diagnosis (determination) result that no tumor exists, but the present embodiment is not limited thereto. Similarly to the above description, the determination function 552 may sequentially perform fourth determination, fifth determination, and so on by using trained models for fourth determination, fifth determination, and so on.

Similarly to the case of the second determination model 524, when the examination result of detailed examination of a subject, which is related to a disease type according to the second determination, defies the determination result of the second determination, the selection function 553 may select a third determination model 525 with a reduced weight for an element related to inference of the disease type according to the second determination.

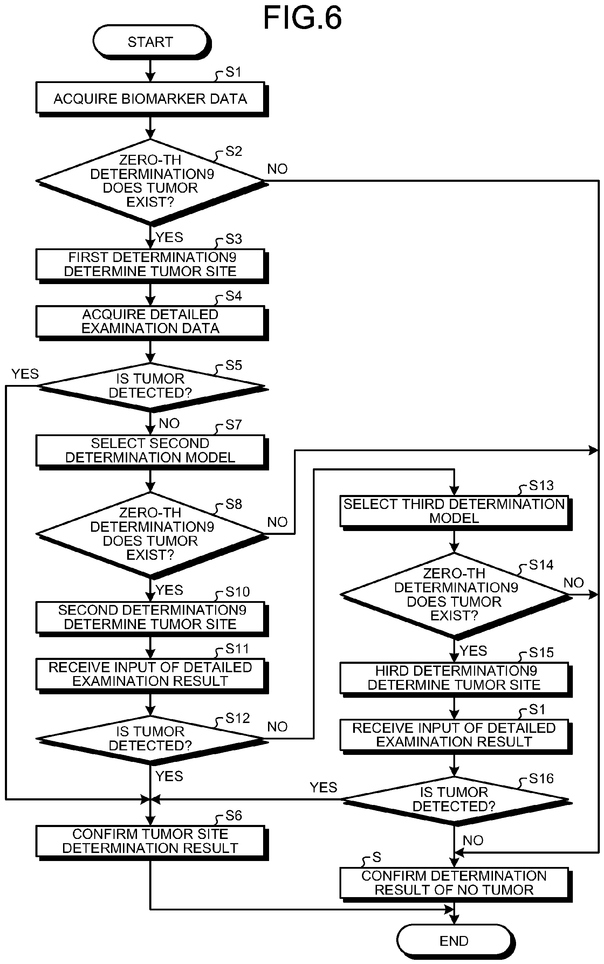

The following describes processing executed by the medical information processing apparatus 5 according to the first embodiment. FIG. 6 is a flowchart illustrating an example of the processing executed by the medical information processing apparatus 5 according to the first embodiment.

First, the control function 551 acquires biomarker data (step S1). Specifically, the control function 551 acquires biomarker data from the sample examination apparatus 2 and stores the biomarker data as the medical examination data 521 in the memory 52.

Subsequently, the determination function 552 performs the zero-th determination and determines the existence of a tumor (cancer) (step S2). Specifically, the determination function 552 inputs the biomarker data acquired by the control function 551 to the zero-th determination model 522 and determines the existence of a cancer based on an output inference result. When it is determined that no cancer exists in the zero-th determination (No at step S2), the determination function 552 confirms a determination result that no tumor exists and ends the present processing (step S6).

When it is determined that a cancer exists in the zero-th determination (Yes at step S2), the determination function 552 inputs the biomarker data acquired by the control function 551 to the first determination model 523. Subsequently, the determination function 552 determines a tumor site based on the inference result of the first determination model 523 (step S3). Thereafter, detailed examination related to the tumor site determined in the first determination is performed on a subject by using the medical image diagnosis apparatus 1 or the like.

Subsequently, the control function 551 receives input of the examination result of the detailed examination after the first determination (step S4). Specifically, the control function 551 receives input of the examination result of the detailed examination, which is input through the terminal apparatus 3 by clinical laboratory technician or the like.

Subsequently, the determination function 552 checks whether a tumor is detected based on the examination result of the detailed examination after the first determination (step S5). When a tumor is detected (Yes at step S5), the determination function 552 confirms, as a determination result, the tumor site determined in the first determination and ends the present processing (step S7).

When no tumor is detected (No at step S5), the selection function 553 selects a second determination model 524 based on the result of the first determination and the examination result of the detailed examination (step S8). Subsequently, similarly to step S2, the determination function 552 performs the zero-th determination and determines the existence of a tumor (cancer) (step S9).

When it is determined that no cancer exists in the zero-th determination (No at step S9), the determination function 552 proceeds to processing at step S6.

When it is determined that a cancer exists in the zero-th determination (Yes at step S9), the determination function 552 performs inputting to the second determination model 524 selected by the selection function 553. Then, the determination function 552 determines a tumor site based on the inference result of the second determination model 524 (step S10). Thereafter, detailed examination related to the tumor site determined in the second determination is performed on the subject by using the medical image diagnosis apparatus 1 or the like.

Subsequently, the control function 551 receives input of the examination result of the detailed examination after the second determination (step S11). Subsequently, the determination function 552 checks whether a tumor is detected based on the examination result of the detailed examination after the second determination (step S12). When a tumor is detected (Yes at step S12), the determination function 552 proceeds to processing at step S7.

When no tumor is detected (No at step S12), the selection function 553 selects a third determination model 525 based on the result of the second determination and the examination result of the detailed examination (step S13). Subsequently, similarly to step S2, the determination function 552 performs the zero-th determination and checks the existence of a tumor (step S14). When it is determined that no cancer exists in the zero-th determination (No at step S14), the determination function 552 proceeds to processing at step S6.

When it is determined that a cancer exists in the zero-th determination (Yes at step S14), the determination function 552 performs inputting to the third determination model 525 selected by the selection function 553. Then, the determination function 552 determines a tumor site based on the inference result of the third determination model 525 (step S15). Thereafter, detailed examination related to the tumor site determined in the third determination is performed on the subject by using the medical image diagnosis apparatus 1 or the like.

Subsequently, the control function 551 receives input of the examination result of the detailed examination after the third determination (step S16). Then, the determination function 552 checks whether a tumor is detected based on the examination result of the detailed examination after the third determination (step S17). When a tumor is detected (Yes at step S17), the determination function 552 proceeds to processing at step S7. When no tumor is detected (No at step S17), the determination function 552 proceeds to processing at step S6.

As described above, the medical information processing apparatus 5 according to the first embodiment performs the first determination of tumor existence and a tumor incidence site by using the first determination model 523 subjected to learning with biomarker data of all subjects, and when no tumor is detected in detailed examination for the site, the medical information processing apparatus 5 performs the second determination by using a second determination model 524 subjected to learning without biomarker data of a subject with a tumor developed at the site.

Accordingly, the second determination model 524 subjected to learning without biomarker data of a subject, which is thought to be the cause of falsely determination in the first determination, can be used in the second determination, and the accuracy of determining a tumor incidence site is expected to improve. With the improvement of the accuracy of determining a tumor incidence site, the number of times of detailed examination performed on a subject can be reduced. Thus, with the medical information processing apparatus 5 according to the first embodiment, it is possible to reduce the load of disease determination on a subject.

### First modification of first embodiment

In the first embodiment described above, when detailed examination detects no tumor at a tumor site determined in the first determination, the second determination is performed by using a model subjected to learning with data except for data of a patient having a tumor at the site. However, a model used in the second determination is not limited thereto.

The present modification describes a case in which, for a tumor site determined in the first determination, the second determination is performed by using a model subjected to learning without biomarker data having high contribution to the determination. The following description is mainly made on a difference from the above-described embodiment, and detailed description is omitted for features common to an already described content. Modification described below may be individually performed or may be performed in combination as appropriate.

The following describes a second determination model 524 of the present modification. The second determination model 524 of the present modification is a trained model subjected to learning with an examination value of a kind of biomarker except for an examination value of a kind of biomarker related to a disease of a disease type determined in the first determination among the examination values of a plurality of kinds of biomarker collected from a plurality of subjects.

More specifically, the second determination model 524 according to the present modification is a trained model subjected to machine learning by inputting, to the machine learning engine as learning data, "biomarker data (protein)", "biomarker data (gene)", ... except for biomarker data having a high specificity for one particular disease (cancer) and "tumor incidence site", the trained model being configured to output the numerical value indicating the probability of being affected as an inference result for each tumor incidence site in accordance with the input of biomarker data.

The biomarker having a high specificity for a particular cancer is, for example, FGFR1 gene, ESR1 gene, or the like detected as blood ctDNA in a case of breast cancer, NRAS gene, FBXW7 gene, or the like detected as ctDNA in a case of colorectal cancer, or MET gene, ALK gene, or the like detected as ctDNA in a case of lung cancer.

For example, when the first determination by the determination function 552 provides a result that the probability of "breast cancer" is high and no "breast cancer" is detected in detailed examination of "breast cancer", the selection function 553 selects, as the second determination model 524, a trained model subjected to learning with data except for data of a biomarker specific to breast cancer, such as FGFR1 gene or ESR1 gene detected as ctDNA. Then, the determination function 552 performs the second determination by using the second determination model 524.

Since the second determination model 524 subjected to learning with data except for data of a biomarker specific to breast cancer is subjected to learning without biomarker data having high contribution to the determination result "breast cancer" of the first determination, the probability that "breast cancer" is determined in the second determination decreases.

Moreover, since the second determination model 524 is a trained model subjected to learning with data except for biomarker data having high contribution to false determination in the first determination, the accuracy of determination in the second determination is expected to improve.

Accordingly, similarly to the first embodiment, with the medical information processing apparatus 5 according to the present modification, the accuracy of the second determination of a tumor incidence site is expected to improve, and thus the number of times of detailed examination performed on a subject can be reduced. Accordingly, with the medical information processing apparatus 5 according to the present modification, it is possible to reduce the load of disease determination on a subject.

### Second modification of first embodiment

In the modification described above, for example, when "breast cancer" is falsely determined in the first determination, the selection function 553 selects a second determination model 524 subjected to learning with data except for data of a biomarker specific to breast cancer.

However, biomarker data of a patient of "breast cancer" is included in the learning data of the second determination model 524, and thus the probability that "breast cancer" is falsely determined in the second determination cannot be eliminated.

Thus, in the present modification, a trained model subjected to learning with data except for biomarker data of a patient of a particular cancer and data of a biomarker specific to the cancer is used as the second determination model 524.

According to the present modification, for example, when "breast cancer" is falsely determined in the first determination, a trained model subjected to learning with data except for data of a patient of "breast cancer" and data of a biomarker specific to "breast cancer", which are thought to be the cause of the false determination in the first determination, is used as the second determination model 524, and thus the accuracy of determination in the second determination is expected to further improve.

### Second embodiment

In the first embodiment described above, a trained model used in the first determination is different from trained models used in the second determination and later determination. A second embodiment describes a case in which the trained model used in the first determination is identical to the trained models used in the second determination and later determination. The following description is mainly made on a difference from the above-described embodiment, and detailed description is omitted for features common to the already described content. Embodiment described below may be individually performed or may be performed in combination as appropriate.

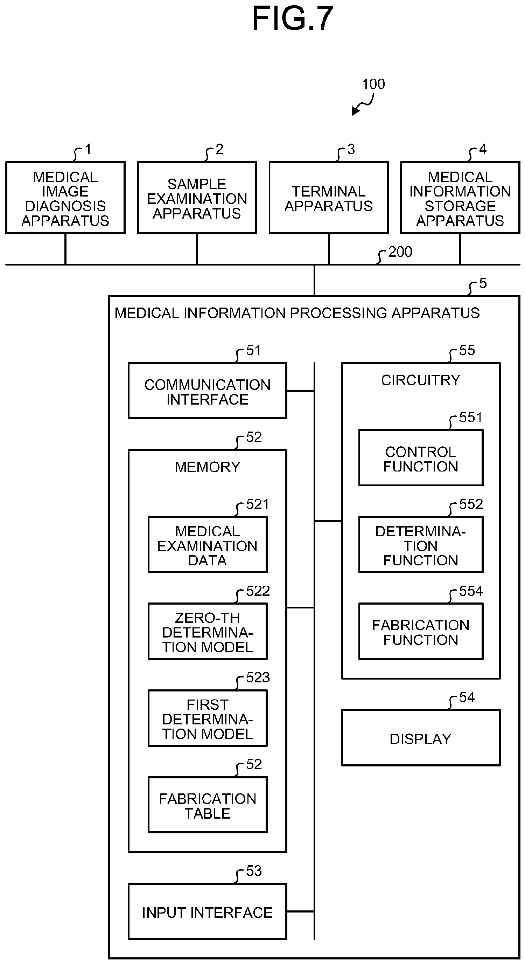

FIG. 7 is a block diagram illustrating an example of the configuration of the medical information processing apparatus 5 according to the second embodiment. The medical information processing apparatus 5 according to the second embodiment is different from that of the first embodiment in that no second determination model 524 nor third determination model 525 is stored in the memory 52, a fabrication table 526 is stored in the memory 52, the processing circuitry 55 has no selection function 553, and the processing circuitry 55 has a fabrication function 554.

The fabrication table 526 is a data table for fabricating biomarker data input to the first determination model 523 at the second determination and the third determination. The fabrication table 526 is, for example, a data table that associates a tumor incidence site, a biomarker specific to the tumor incidence site, and the upper limit value, lower limit value, or standard value of the biomarker.

Specifically, the fabrication table 526 is a data table that associates a tumor incidence site, a biomarker specific to the tumor incidence site, and the upper or lower limit value of the biomarker, such as "breast cancer", "CA15-3", and "0 U/mL". There may be a plurality of biomarkers specific to a tumor incidence site.

When the examination result of detailed examination after the first determination defies the determination result, the fabrication function 554 provides change in accordance with a disease type determined in the first determination to the examination values of a plurality of kinds of biomarkers collected from subjects.

For example, when the examination result of detailed examination after the first determination defies the determination result, the fabrication function 554 invalidates the examination value of a kind of biomarker related to a disease of a disease type determined in the first determination among the examination values of a plurality of kinds of biomarkers collected from subjects.

Specifically, the fabrication function 554 fabricates biomarker data of a subject based on the determination result of the first determination by the determination function 552 and the fabrication table 526. For example, when the first determination by the determination function 552 provides a result that the probability of "breast cancer" is high and no "breast cancer" is detected in detailed examination of "breast cancer", the fabrication function 554 refers to the fabrication table 526 and specifies a biomarker (in the above-described example, CA15-3) specific to "breast cancer".

Subsequently, the fabrication function 554 converts the biomarker data of the subject for the specified biomarker into a lower limit value (in the above-described example, "0 U/mL") associated in the fabrication table 526.

In this example, the fabrication function 554 changes, to the lower limit value, the biomarker data related to the tumor site ("breast cancer") determined in the first determination, but the fabrication function 554 may convert the biomarker data into the upper limit value. This is because the amount of a material normally existing in blood decreases due to tumor development and the material becomes no longer detected in some cases. The biomarker data may be changed to the standard value, in other words, the amount of a material existing in blood may be changed to the value of a healthy subject (such as the median, upper limit value, or lower limit value of the range of healthy values).

The fabrication function 554 may delete the biomarker data related to the tumor site ("breast cancer") determined in the first determination. Conversion of the biomarker data into the upper limit value or the lower limit value or deletion of the biomarker data is an example of examination value invalidation.

The determination function 552 inputs the biomarker data converted by the fabrication function 554 to the first determination model 523 and performs the second determination. It is thought that influence of biomarker data as the cause of the determination result "breast cancer" of the first determination can be eliminated by fabricating data of a biomarker specific to breast cancer into the upper limit value, the lower limit value, or the standard value. Thus, the accuracy of determination in the second determination is expected to improve.

When the examination results of detailed examination after the second determination and later determination defy the determined disease type, the determination function 552 newly determines a disease type based on an inference result obtained by inputting, to the first determination model 523, an examination value obtained by invalidating the examination values of kinds of biomarkers related to all disease types determined so far among the examination values of a plurality of kinds of biomarkers collected from subjects.

For example, when the control function 551 receives input of an examination result that no tumor is detected in detailed examination after the second determination, the fabrication function 554 performs the third determination through the same processing as in the second determination. Specifically, when the first determination provides a result that the probability of "breast cancer" is high, the second determination provides a result that the probability of "colorectal cancer" is high, and none of the cancers are detected in detailed examination, the fabrication function 554 refers to the fabrication table 526, specifies a biomarker specific to "breast cancer" and a biomarker specific to "colorectal cancer", and converts each specified biomarker in to the upper limit value, lower limit value, or standard value thereof.

In the present embodiment, when no tumor is detected in detailed examination after the third determination, the determination function 552 confirms a diagnosis (determination) result that no tumor exists, but the present embodiment is not limited thereto. Similarly to the above description, the determination function 552 may sequentially perform fourth determination, fifth determination, and so on.

In the present embodiment, when the examination result of detailed examination after the first determination defies the determination result, the fabrication function 554 invalidates the examination value of a kind of biomarker related to a disease of a disease type determined in the first determination among the examination values of a plurality of kinds of biomarkers collected from subjects, but the method of fabricating biomarker data is not limited thereto.

For example, the fabrication function 554 may perform fabrication with which an examination value of a kind of biomarker related to a disease of a disease type determined in the first determination is made closer to a reference value by, for example, multiplying the examination value by a coefficient smaller than one (or coefficient equal to or larger than one).

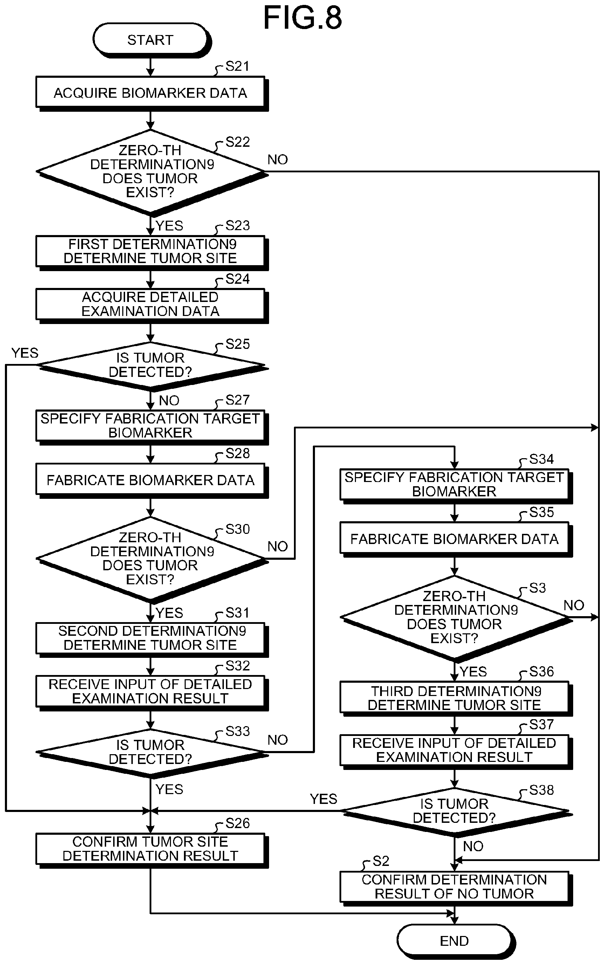

The following describes processing executed by the medical information processing apparatus 5 according to the second embodiment. FIG. 8 is a flowchart illustrating an example of the processing executed by the medical information processing apparatus 5 according to the second embodiment.

Steps S21 to S27 are the same processing as steps S1 to S7 in FIG. 6, and thus description thereof is omitted.

The fabrication function 554 refers to the fabrication table 526 and specifies a fabrication target biomarker (step S28). For example, when the first determination provides a result that the probability of "breast cancer" is high and no "breast cancer" is detected in detailed examination of "breast cancer", the fabrication function 554 refers to the fabrication table 526 and specifies a biomarker specific to "breast cancer".

Subsequently, the fabrication function 554 converts (fabricates) the biomarker data of the subject for the specified biomarker into the upper or lower limit value thereof associated in the fabrication table 526 (step S29). The determination function 552 inputs the biomarker data of the subject, which is fabricated by the fabrication function 554, to the first determination model 523.

Steps S30 to S33 are the same processing as steps S9 to S12 in FIG. 6, and thus description thereof is omitted. In addition, steps S34 to S35 are the same processing as steps S28 to S29, and thus description thereof is omitted. In addition, steps S36 to S39 are the same processing as steps S14 to S17 in FIG. 6, and thus description thereof is omitted.

The medical information processing apparatus 5 according to the second embodiment performs the first determination of tumor existence and a tumor incidence site by using the first determination model 523 subjected to learning with biomarker data collected from a plurality of subjects, and when the control function 551 receives input of an examination result that no tumor is detected in detailed examination for the site, the medical information processing apparatus 5 converts the biomarker data of a biomarker specific to the site into the upper limit value, the lower limit value, or the standard value thereof and then inputs the converted biomarker data to the first determination model 523 and performs the second determination.

Accordingly, no trained model different from the trained model used in the first determination needs to be prepared in the second determination and later determination, and thus the load of processing of generating a trained model can be reduced. Moreover, since biomarker data thought to be the cause of false determination in the first determination is converted into a value having no influence on determination, the cause of the false determination is thought to be eliminated and the accuracy of determination in the second determination is expected to improve.

Accordingly, similarly to the first embodiment, with the medical information processing apparatus 5 according to the second embodiment, the accuracy of the second determination of a tumor incidence site is expected to improve, and thus the number of times of detailed examination performed on a subject can be reduced. Accordingly, with the medical information processing apparatus 5 according to the second embodiment, it is possible to reduce the load of processing of generating a learning model and reduce the load of disease determination on a subject.

In each embodiment described above, the determination function 552 determines a tumor incidence site as a disease type, but the embodiments are not limited thereto. For example, the determination function 552 may determine myocardial infarction or angina as the disease type of a cardiac disease, and chronic obstructive lung disease or interstitial lung disease as the disease type of a lung disease.

In each embodiment described above, an acquisition unit, a determination unit, and a reception unit in the present specification are achieved by the control function 551, the determination function 552, the selection function 553, and the fabrication function 554 of the processing circuitry 55, but the embodiments are not limited thereto. For example, the acquisition unit, the determination unit, and the reception unit in the present specification may be achieved by hardware only or by mixture of hardware and software instead of being achieved by the control function 551, the determination function 552, the selection function 553, and the fabrication function 554 described in the embodiment(s).

When the processing circuitry 55 is achieved by a processor as described above, each processing function included in the processing circuitry 55 is stored in the memory 52 in the form of computer-executable program. The processing circuitry 55 reads computer programs from the memory 52 and executes the computer programs, thereby achieving a function corresponding to each computer program. In other words, the processing circuitry 55 having read a computer program has the corresponding function illustrated in the processing circuitry 55 in FIG. 1.

In FIG. 1, the processing functions are implemented by a single processor, but the processing circuitry may be configured by combining a plurality of independent processors and each processor may execute a computer program to implement the corresponding function. The processing functions of the processing circuitry 55 may be integrated or distributed into one piece or a plurality of pieces of circuitry as appropriate.

In the example illustrated in FIG. 1, each computer program corresponding to a processing function is stored in the single memory 52, but a plurality of memories may be arranged in a distributed manner and each computer program may be read from the corresponding memory by the processing circuitry.

The term "processor" used in the above description means a circuit such as a central processing unit (CPU), a graphics processing unit (GPU), an application specific integrated circuit (ASIC), or a programmable logic device (for example, a simple programmable logic device (SPLD), a complex programmable logic device (CPLD), or a field programmable gate array (FPGA)).

The processor implements a function by reading and executing a computer program stored in the memory 52. The computer program may be directly incorporated in a circuit of the processor instead of being stored in the memory 52. In this case, the processor implements the function by reading and executing the computer program incorporated in the circuit. The processor of each above-described embodiment is not limited to a configuration in which the processor is configured as a single circuit, but may be configured as one processor by combining a plurality of independent circuits to implement the function of the processor.

A computer program executed by the processor is incorporated in a read-only memory (ROM), a memory, or the like in advance and provided. The computer program may be recorded and provided in a computer-readable storage medium such as a CD (Compact Disc)-ROM, a flexible disk (FD), CD-R (Recordable), or a digital versatile disc (DVD) as a file in a format installable or executable on the apparatuses.

The computer program may be stored in a computer connected to a network such as the Internet and provided or distributed by being downloaded through the network. For example, the computer program is configured as a module including the above-described functional units. In an actual hardware configuration, the module is loaded onto a main storage apparatus and generated on the main storage apparatus as a CPU reads and executes the computer program from a storage medium such as a ROM.

According to at least one embodiment described above, it is possible to reduce the load of disease determination on a subject.

While certain embodiments have been described, these embodiments have been presented by way of example only, and are not intended to limit the scope of the inventions. Indeed, the novel embodiments described herein may be embodied in a variety of other forms; furthermore, various omissions, substitutions and changes in the form of the embodiments described herein may be made without departing from the scope of the invention as defined by the appended claims. The accompanying claims are intended to cover such forms or modifications as would fall within the scope of the invention.

## Claims

1. A medical information processing apparatus (5) comprising:
an acquisition unit (551) configured to acquire an examination value of a biomarker collected from an examination target subject;
a determination unit (552) configured to determine a first disease type of a disease with which the subject is potentially affected based on an inference result of a first trained model (523), the inference result being obtained by inputting the examination value acquired by the acquisition unit to the first trained model (523), the first trained model (523) being configured to infer the first disease type of an affected disease upon inputting of the examination value; and
a reception unit (551) configured to receive input of an examination result related to the first disease type of the subject and obtained through examination by a method different from the biomarker, wherein
the determination unit (552, 553) is configured to determine, when the examination result of the subject defies the first disease type, a second disease type of the disease with which the subject is potentially affected by using a second trained model (524) in accordance with the defied first disease type.

2. The medical information processing apparatus (5) according to claim 1, wherein the determination unit (552, 553) determines the second disease type of the disease with which the subject is potentially affected by using the second trained model (524) obtained by excluding an element related to inference of the first disease type from the first trained model (523).

3. The medical information processing apparatus (5) according to claim 1, wherein the second trained model (524) is a trained model which has been trained with examination values which exclude examination values of a subject affected with a disease of the first disease type among examination values of biomarkers collected from a plurality of subjects.

4. The medical information processing apparatus (5) according to claim 1, wherein the second trained model (524) is a trained model which has been trained with examination values of a plurality of kinds of biomarkers collected from a plurality of subjects excluding examination values of a kind of biomarker corresponding to a disease of the first disease type.

5. The medical information processing apparatus (5) according to claim 1, wherein when the examination result of the subject defies the second disease type, the determination unit (552) newly determines the second disease type by using a trained model obtained by excluding an element related to inference of the second disease type from the trained model used to determine the second disease type.

6. The medical information processing apparatus (5) according to claim 1, wherein
the determination unit (552) is configured to determine whether the subject is affected with the disease based on an inference result of a third trained model (522), the inference result being obtained by inputting the examination value acquired by the acquisition unit (551) to the third trained model, the third trained model being configured to output existence of the disease as an inference result upon inputting of the examination value, and
when having determined that the subject is affected with the disease, the determination unit (552) inputs the examination value acquired by the acquisition unit to the first trained model.

7. A medical information processing apparatus (5) comprising:
an acquisition unit (551) configured to acquire an examination value of a biomarker collected from an examination target subject;
a determination unit (552) configured to determine a first disease type of a disease with which the subject is potentially affected based on an inference result of a trained model (523), the inference result being obtained by inputting the examination value acquired by the acquisition unit (551) to the trained model, the trained model (523) being configured to infer the first disease type of an affected disease upon inputting of the examination value; and
a reception unit (551) configured to receive input of an examination result related to the first disease type of the subject and obtained through examination by a method different from the biomarker, wherein
the determination unit (552, 554) is configured to determine, when the examination result of the subject defies the first disease type, a second disease type of the disease with which the subject is potentially affected based on an inference result obtained by providing change in accordance with the defied first disease type to examination values of a plurality of kinds of biomarkers collected from the subject and inputting the examination values provided with the change to the trained model.

8. The medical information processing apparatus (5) according to claim 7, wherein the determination unit (552, 554) is configured to apply the change by invalidating an examination value of a kind of biomarker related to a disease of the defied first disease type among examination values of a plurality of kinds of biomarkers collected from the subject.

9. The medical information processing apparatus (5) according to claim 7, wherein when the examination result of the subject defies the second disease type, the determination unit (552, 554) newly determines the second disease type based on an inference result obtained by inputting, to the trained model, an examination value obtained by invalidating an examination value of a kind of biomarker related to diseases of the first disease type and the second disease type among examination values of a plurality of kinds of biomarkers collected from the subject.

10. The medical information processing apparatus (5) according to any one of claims 1 to 9, wherein the disease is cancer.

11. A computer readable medium comprising instructions that cause a computer to execute:
acquiring an examination value of a biomarker collected from an examination target subject;
determining a first disease type of a disease with which the subject is potentially affected based on an inference result of a first trained model, the inference result being obtained, the first trained model being configured to infer the first disease type of an affected disease upon inputting of the examination value;
receiving input of an examination result related to the first disease type of the subject and obtained through examination by a method different from the biomarker; and
determining, when the examination result of the subject defies the first disease type, a second disease type of the disease with which the subject is potentially affected by using a second trained model in accordance with the defied first disease type.

12. A computer readable medium comprising instructions that cause a computer to execute:
acquiring an examination value of a biomarker collected from an examination target subject;
determining a first disease type of a disease with which the subject is potentially affected based on an inference result of a first trained model, the inference result being obtained by inputting the acquired examination value at the acquiring to the first trained model, the first trained model being configured to infer the first disease type of an affected disease upon inputting of the examination value;
receiving input of an examination result related to the first disease type of the subject and obtained through examination by a method different from the biomarker; and
determining, when the examination result of the subject defies the first disease type, a second disease type of the disease with which the subject is potentially affected based on an inference result obtained by providing change in accordance with the defied first disease type to examination values of a plurality of kinds of biomarkers collected from the subject and inputting the examination values provided with the change to the first trained model.
